# EUROPEAN PATENT SPECIFICATION

(11) **EP 0 787 501 B1**
(45) Date of publication and mention of the grant of the patent: **09.04.2003**
(21) Application number: 97400147.1
(22) Date of filing: 23.01.1997
(51) Int. Cl.: A61M 5/24, A61M 5/32, A61M 5/34

(54) **Injector**
Injektor
Injecteur

(30) Priority: 23.01.1996 JP 2998596
(43) Date of publication of application: 06.08.1997
(73) Proprietor: TERUMO KABUSHIKI KAISHA, Tokyo 151 (JP)
(72) Inventor: Fukuda, Masaki, c/o Terumo Kabushiki Kaisha, Nakakoma-gun, Yamanashi-ken 409-38 (JP); Matsuki, Tetsuya, c/o Terumo Kabushiki Kaisha, Nakakoma-gun, Yamanashi-ken 409-38 (JP)
(74) Representative: Joly, Jean-Jacques

(56) References cited:
- EP-A- 0 140 995
- EP-A- 0 206 582
- EP-A- 0 364 777
- WO-A-92/04926
- WO-A-94/21313
- FR-A- 2 563 436
- US-A- 3 838 690

## Description

### BACKGROUND OF THE INVENTION

### Field of the Invention:

The present invention relates to an injector of the type which houses a medicament solution cartridge, such as a dental injector, a double-headed injection needle for use in such an injector, and an injection needle disposal container for removing an injection needle from an injector and holding the removed injection needle.

### Description of the Related Art:

Heretofore, there have been reusable dental injectors made of a sterilizable material such as metal and combined with detachable medicament solution cartridges. Such a reusable dental injector has a needle hub attachment which is usually externally threaded. An injection needle which can be used on the injector comprises a double-headed hollow needle having front and rear pointed ends and a needle hub fixedly mounted on an intermediate portion of the double-headed hollow needle and an open rear end. The open rear end of the needle hub is internally threaded for being threaded over the externally threaded needle hub attachment. When the needle hub is threaded over the needle hub attachment, the injection needle is securely coupled to the injector. A medicament solution cartridge to be mounted in the injector comprises a closure member on a front end thereof which can be pierced by the rear pointed end of the injection needle and a gasket slidable toward a rear end of the medicament solution cartridge. The medicament solution cartridge is filled with a solution of a medicament such as a dental anesthetic. The injector has a plunger for pushing the gasket of the medicament solution cartridge which is housed in the injector.

For attaching the injection needle to the injector, it is necessary to rotate the needle hub relatively to the injector. After the injection needle has been used, it has to be detached from the injector because the injector itself is reused. Therefore, the needle hub also needs to be rotated relatively to the injector for detachment of the injection needle. Accordingly, the injection needle cannot easily be attached to and detached from the injector.

In dental use, the dentist is often required to intentionally curve or bend the injection needle in order to administer an injection within a small space in the mouth of the patient. Since it is difficult to return the injection needle which has been curved or bent once back to its original shape, the dentist frequently finds it necessary to replace the curved or bent injection needle with a new injection needle while the same medicament solution cartridge is still in use. The process of changing injection needles is not easy to carry out because of the threaded installation of the needle hub on the needle hub attachment.

EP-A-0 364 777 discloses an injector comprising a medicament solution cartridge container and an injection needle attachment mounted on the medicament solution cartridge container. An injection needle is detachably mounted on the injection needle attachment. The injection needle is hollow and has front and rear piercing portions and a needle hub in the middle. The injection needle attachment comprises a hub holder holding the needle hub, a movable assembly, and a hub attaching and detaching mechanism for releasing the needle hub from the hub holder when the movable assembly moves towards the rear end of the injector and holding the needle hub when the movable assembly moves towards a front end of the injector.

### SUMMARY OF THE INVENTION

It is a general object of the present invention to provide an injector which allows a medicament solution cartridge to be easily attached thereto and detached therefrom, and which can be reused.

A major object of the present invention is to provide an injection needle of double-headed design which can easily be attached to and detached from an injector without relying on threaded engagement or disengagement between a needle hub and a hub holder.

The present invention is defined in appended claims 1 to 13.

The above and other objects, features, and advantages of the present invention will become apparent from the following description when taken in conjunction with the accompanying drawings in which preferred embodiments of the present invention are shown by way of example.

### BRIEF DESCRIPTION OF THE DRAWINGS

FIG. 1 is a plan view of an injector according to the present invention with an injection needle attached thereto;
FIG. 2 is an enlarged fragmentary cross-sectional view of a front end portion of the injector with the injection needle attached thereto;
FIG. 3 is a plan view of a hub holder of an injection needle attachment of the injector shown in FIG. 2;
FIG. 4 is a front elevational view of the hub holder shown in FIG. 3;
FIG. 5 is a plan view of a movable assembly of the injection needle attachment of the injector shown in FIG. 2;
FIG. 6 is a cross-sectional view taken along line VI - VI of FIG. 5;
FIG. 7 is a front elevational view, partly in cross section, of a needle hub for use on an injection needle according to the present invention;
FIG. 8 is a bottom view of the needle hub shown in FIG. 7;
FIG. 9 is an enlarged fragmentary cross-sectional view showing the manner in which the injection needle is detached from the injector;
FIG. 10 is an enlarged fragmentary cross-sectional view of a hub holder of an injector according to another embodiment of the present invention, showing a finger held in engagement with a flange of a needle hub;
FIG. 11 is an enlarged fragmentary cross-sectional view similar to FIG. 10, showing the finger brought out of engagement with the flange of the needle hub; and
FIG. 12 is a cross-sectional view of an injection needle disposal container according to the present invention.

### DETAILED DESCRIPTION OF THE PREFERRED EMBODIMENTS

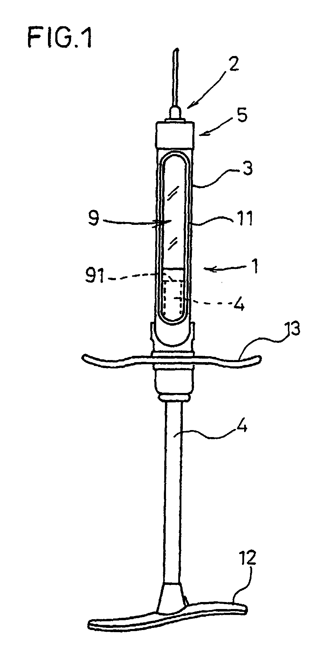

As shown in FIG. 1, an injector 1 according to the present invention comprises a medicament solution cartridge container 3 for housing a medicament solution cartridge 9, a plunger 4 for pushing a gasket 91 in the medicament solution cartridge 9, and an injection needle attachment 5.

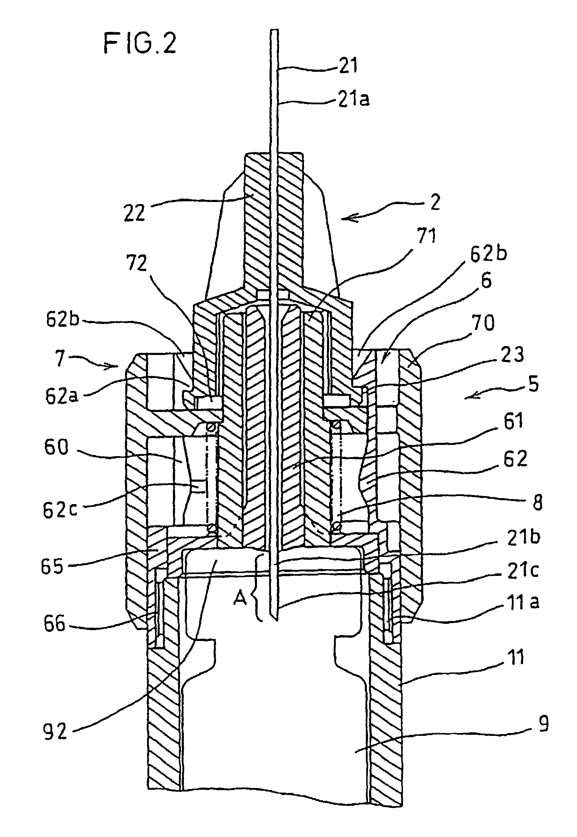

As shown in FIG. 2, the injection needle attachment 5 comprises a hub holder 6 for holding a needle hub 22 on an injection needle 2, a movable assembly 7 movable toward a rear end of the injector 1 remote from the injection needle 2, and a hub attaching and detaching mechanism (injection needle attaching and detaching mechanism) for releasing the needle hub 22 from the hub holder 6 when the movable assembly 7 moves toward the rear end of the injector 1 and holding the needle hub 22 with the hub holder 6 when the movable assembly 7 moves toward a front end of the injector 1, i.e., toward the injection needle 2.

The injection needle 2 comprises a hollow needle 21 having front and rear pointed ends and the needle hub 22 fixedly mounted on an intermediate portion of the hollow needle 21 and having an open rear end.

As shown in FIG. 1, the injector 1 also has an injector casing 11 with the medicament solution cartridge container 3 disposed therein, a plunger presser 12 fixed to a rear end of the plunger 4 which extends out of a rear end of the injector casing 11, and a finger grip 13 mounted on the rear end of the injector casing 11 for being engaged by a finger. In FIG. 1, the tip end of the plunger 4 is shown as entering the medicament solution cartridge 9 and contacting the gasket 91. When the plunger 4 is pulled backward away from the injector casing 11, the medicament solution cartridge 9 can be removed from the medicament solution cartridge container 3.

The injection needle attachment 5 and the injection needle attaching and detaching mechanism will be described in specific detail.

The injection needle attachment 5, which is mounted on a front end of the injector casing 11, has a structure as shown in FIG. 2. In FIG. 2, the injection needle 2 is shown as being mounted on the injection needle attachment 5. The rear pointed end, denoted by 21c, of the hollow needle 21 pierces a sealing member 92 which closes a front end of the medicament solution cartridge 9 for discharging a solution of a medicament contained in the medicament solution cartridge 9.

The injection needle attachment 5 allows the needle hub 22 to be easily attached thereto and detached therefrom without relying on threaded engagement or disengagement. Accordingly, the injection needle 2 can quickly be mounted on and dismounted from the injector 1.

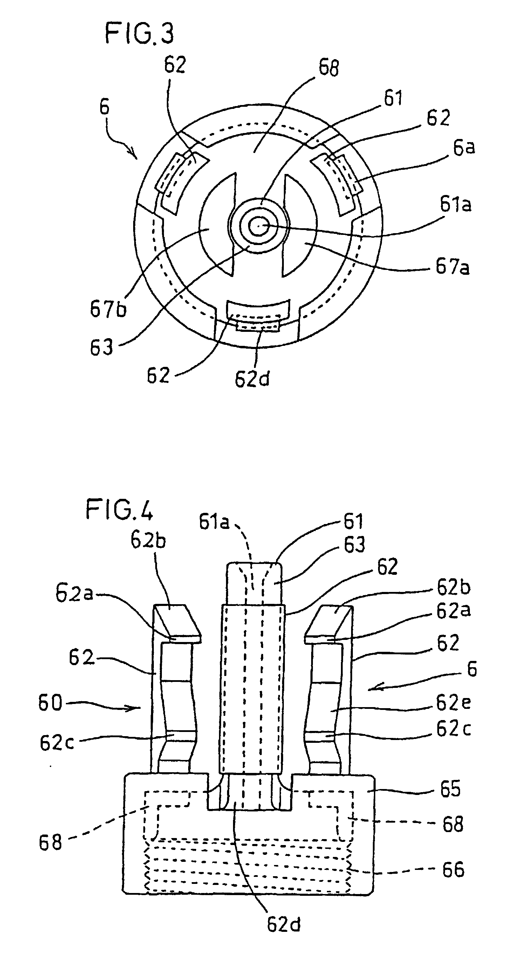

As shown in FIGS. 3 and 4, the hub holder 6 comprises a hub holder finger assembly 60 and a tubular guide member 61 for guiding a cartridge piercing portion 21b of the hollow needle 21. The hub holder finger assembly 60 and the tubular guide member 61 are fixed to each other. The hub holder finger assembly 60 and the tubular guide member 61 may be of an integral unitary structure.

The hub holder finger assembly 60 comprises a cylindrical base 65 and a plurality of (three in the illustrated embodiment) hub holder fingers 62 mounted at equal angular intervals on an upper surface of the cylindrical base 65. Each of the hub holder fingers 62 comprises a hub guiding slanted surface 62b disposed on an upper distal end thereof, a flange engaging edge 62a disposed on a lower terminal end of the hub guiding slanted surface 62b for engaging a radially outward flange 23 (see FIG. 2) of the needle hub 22, an inwardly projecting edge 62c spaced downwardly from the flange engaging edge 62a for elastically deforming the hub holder finger 62 radially outwardly with respect to the cylindrical base 65, a second slanted surface 62e disposed forward of the inwardly projecting edge 62c and extending therefrom toward the flange engaging edge 62a, and an elastically deformable neck 62d extending downwardly from the inwardly projecting edge 62c and joined to the cylindrical base 65 for allowing the hub holder finger 60 to be easily elastically deformed radially outwardly and spring back radially inwardly. The cylindrical base 65 has an internally threaded inner surface 66 which is threaded over an externally threaded outer surface 11a (see FIG. 2) of the front end of the injector casing 11.

The tubular guide member 61 comprises a disk 68 fixed to the hub holder finger assembly 60 and a tubular body 63 projecting upwardly from a central portion of the disk 68. The disk 68 has two substantially crescent-shaped diametrically opposite recesses 67a, 67b defined therein near the proximal end of the tubular body 63. The tubular body 63 has a guide passage 61a defined axially therein for guiding therethrough the cartridge piercing portion 21b of the hollow needle 21.

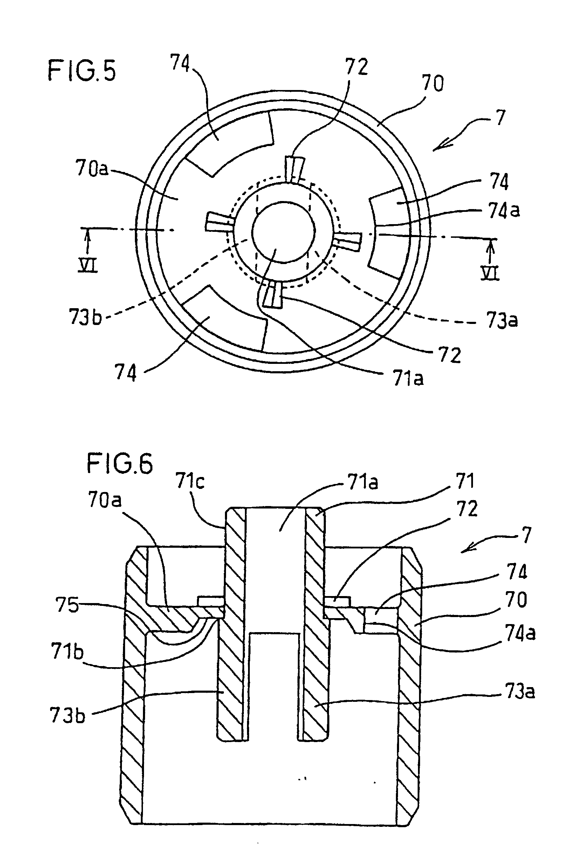

As shown in FIGS. 5 and 6, the movable assembly 7 comprises an outer tubular member 70 and an inner tubular member 71 fixedly mounted concentrically on a central portion of the outer tubular member 70. The outer tubular member 70 has a radial planar panel 70a disposed at a position which is displaced slightly from the center toward front end of the outer tubular member 70. The planar panel 70a has a central opening in which the inner tubular member 71 is axially inserted. The planar panel 70a also has a plurality of (three in the illustrated embodiment) recesses 74 defined at equal angular intervals in an outer circumferential edge thereof. The hub holder fingers 62 extend respectively through the recesses 74.

The hub holder finger assembly 60 may have two or four or more hub holder fingers 62, and the movable assembly 7 may have two or four or more recesses 74. Preferably, the hub holder finger assembly 60 has three hub holder fingers 62 and the movable assembly 7 has three recesses 74, as illustrated, for advantages with respect to the manufacturing process and desired mechanical strength.

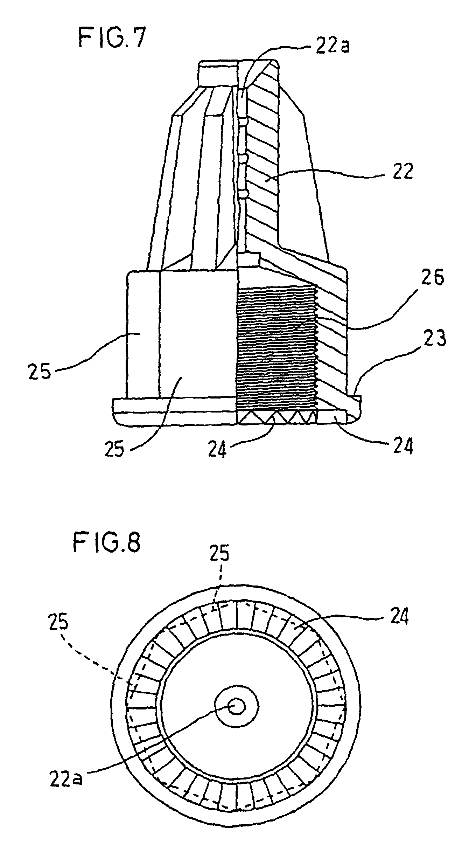

The injection needle attachment 5 has a needle rotation stop for contacting the needle hub 22 to prevent the injection needle 2 from being rotated when the injection needle 2 is mounted on the injector 1. Specifically, the needle rotation stop comprises a plurality of ribs 72 mounted on an upper surface of the planar panel 70a around the central opening therein, i.e., around the inner tubular member 71. The ribs 72 can engage in rotation stop grooves 24 (see FIG. 7) of the needle hub 22 as described later on. Each of the ribs 72 has a triangular cross-sectional shape taken transversely to the longitudinal direction thereof and extends substantially perpendicularly to the axis of the movable assembly 7. While the needle rotation stop may have only one rib 72, it should preferably have a plurality of ribs 72, as shown, for effectively preventing the needle hub 22 from rotating. Specifically, the needle rotation stop should have two to ten ribs 72, and more preferably three to eight ribs 72. The planar panel 70a has an annular recess 75 defined in a lower surface thereof around the central opening therein, i.e., around the inner tubular member 71. The annular recess 75 cooperates with an outer wall surface of the inner tubular member 71 in defining a holder for holding an end of a spring 8 (described later on).

The inner tubular member 71 has a passage 71a defined axially therethrough for insertion therein of the tubular body 63 of the tubular guide member 61. The inner tubular member 71 also has a radial shoulder 71b on a substantially central outer circumferential surface for being joined to the outer tubular member 70, a hub entrance boss 71c extending upwardly from the radial shoulder 71b for entering the needle hub 22 to prevent the needle hub 22 from wobbling when the injection needle 2 is mounted on the injector 1, and a pair of diametrically opposite cartridge pressers 73a, 73b extending downwardly from the radial shoulder 71b for depressing the medicament solution cartridge 9. Each of the cartridge pressers 73a, 73b has a substantially crescent-shaped cross-sectional shape as indicated by the broken lines in FIG. 5. The cartridge pressers 73a, 73b can move downwardly through the respective recesses 67a, 67b shown in FIG. 3. While only one cartridge presser may be employed, the plural (two) cartridge pressers are effective in easily moving the medicament solution cartridge 9. The hub entrance boss 71c is shown as being cylindrical in shape. However, it may have a hollow shape of polygonal cross section. The hub entrance boss 71c is of such a size and shape that it can be inserted into the needle hub 22 without its outer side wall surface engaging the inner surface of the needle hub 22.

As shown in FIG. 2, the injection needle attachment 5 is assembled by placing the spring 8 and the hub holder 6 in the movable assembly 7. The spring 8 comprises a helical spring disposed around the two cartridge pressers 73a, 73b of the movable assembly 7. The three hub holder fingers 62 of the hub holder 6 project from the respective recesses 74 defined in the planar panel 70a of the movable assembly 7. The movable assembly 7 and the hub holder 6 are normally urged to move away from each other by the spring 8 which is interposed therebetween. When the injection needle 2 is not mounted on the injector 1, however, the movable assembly 7 and the hub holder 6 are not separated from each other because the flange engaging edges 62a of the hub holder fingers 62 engage the planar panel 70a. Stated otherwise, the flange engaging edges 62a of the hub holder fingers 62 engage the planar panel 70a for thereby keeping the spring 8 under compression.

When the cartridge piercing portion 21b of the hollow needle 21 is inserted into the guide passage 61a and pushed in, the flange 23 of the needle hub 22 is brought into contact with the hub guiding slanted surfaces 62b of the hub holder fingers 62. Continued pushed movement of the cartridge piercing portion 21b causes the hub holder fingers 62 to spread outwardly elastically. When the flange 23 moves past the flange engaging edges 62a, the hub holder fingers 62 spring back to their original position, whereupon the injection needle 2 is fixedly mounted on the injection needle attachment 5, as shown in FIG. 2. With the injection needle 2 thus mounted in position, the movable assembly 7 is displaced slightly more toward the injector casing 11 than before injection needle 2 is mounted on the injection needle attachment 5. The flange 23 is sandwiched between the flange engaging edges 62a and the planar panel 70a and held in position under repelling forces from the spring 8 which is being compressed.

As shown in FIG. 2, the hollow needle 21 is a double-headed needle having a piercing portion 21a on its front end for piercing the patient and the cartridge piercing portion 21b on its rear end for piercing the sealing member 92 of the medicament solution cartridge 9. As shown in FIGS. 7 and 8, the needle hub 22 has a hollow needle insertion passage 22a defined axially in an end portion thereof. The flange 23 is positioned on an opposite end thereof remote from the hollow needle insertion passage 22a. The rotation stop grooves 24 are defined to radially extend in the end of the flange 23 near the flange 23 in an annular pattern. The rotation stop grooves 24 serve to receive the respective ribs 72 on the planar panel 70a for preventing the injection needle 2 from rotating relatively to the injector 1. The needle hub 22 also includes a cylindrical side wall 25 extending axially between the hollow needle insertion passage 22a and the rotation stop grooves 24. The cylindrical side wall 25 has an internally threaded inner surface 26 for being threaded over another injector of conventional design. The cylindrical side wall 25 is of a polygonal cross-sectional shape, specifically, an octagonal cross-sectional shape, as indicated by the broken lines in FIG. 8, for allowing the user to easily grip the needle hub 22 for rotation.

The injector 1, including the injection needle attachment 5, is made of a material which is sterilizable by an autoclave and somewhat durable, e.g., metal such as stainless steel or a synthetic resin which is hard and heat-resistant.

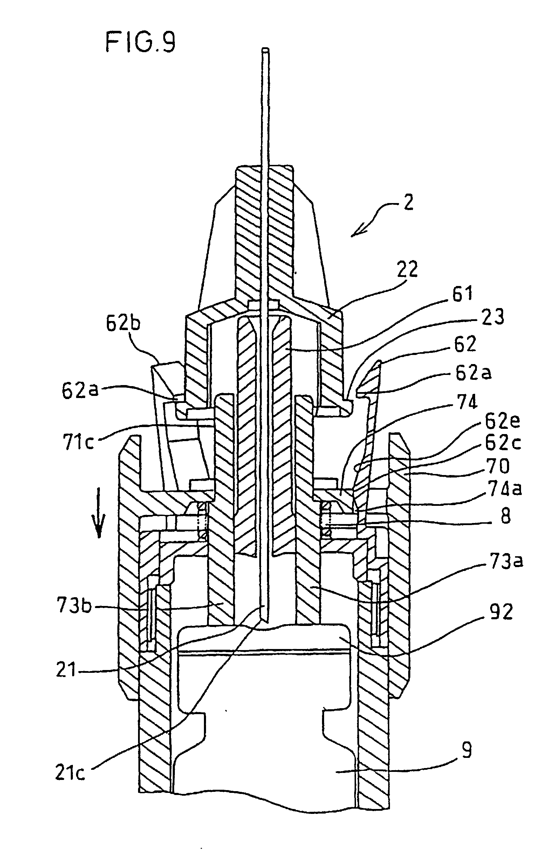

The injection needle attachment 5 can be installed on the injector casing 11, as shown in FIGS. 2 and 9, when the internally threaded inner surface 66 of the cylindrical base 65 of the hub holder 6 is threaded over the externally threaded outer surface 11a of the front end of the injector casing 11. FIG. 9 shows the manner in which the injection needle 2 is detached from the injector 1. The hub holder finger assembly 60 and the tubular guide member 61 fixed thereto are immovable with respect to the injector casing 11. Since, however, the movable assembly 7 is not fixed with respect to the injector casing 11 and the hub holder 6, the movable assembly 7 can be moved toward the rear end of the injector casing 11 away from the injection needle 2 when the movable assembly 7 is pushed toward the rear end of the injector casing 11 in the direction indicated by the arrow in FIG. 9 against the bias of the spring 8. Upon the movement of the movable assembly 7 toward the rear end of the injector casing 11, the recesses 74 defined in the planar panel 70a also move backward along the inner surfaces of the hub holder fingers 62. Inner surfaces 74a of the recesses 74 slidingly contact and gradually push the second slanted surfaces 62e, which are progressively thicker of the hub holder fingers 62 radially outwardly until the inner surfaces 74a contact the inwardly projecting edges 62c whereupon the flange engaging edges 62a are displaced radially outwardly clear out of contact with the flange 23 of the needle hub 22. The flange engaging edges 62a and the flange 23 are now held out of engagement with each other as shown in FIG. 9.

At this time, the inner tubular member 71 fixed to the outer tubular member 70 also moves toward the rear end of the injector casing 11. The cartridge pressers 73a, 73b on the rear end of the inner tubular member 71 move respectively through the recesses 67a, 67b of the hub holder 6 into engagement with the front end of the medicament solution cartridge 9, and then lower the medicament solution cartridge 9 toward the rear end of the injector casing 11. The movable assembly 7 moves toward the rear end of the injector casing 11 by a distance which is greater than a distance A (see FIG. 2) by which the cartridge piercing portion 21b has entered the medicament solution cartridge 9. Therefore, the front end of the sealing member 92 of the medicament solution cartridge 9 is positioned behind the rear pointed end 21c of the hollow needle 21. The cartridge piercing portion 21b is now held out of contact with the medicament solution cartridge 9. At this time, the front end portion of the tubular guide member 61 enters the cylindrical side wall 25 of the needle hub 22 with the cartridge piercing portion 21b inserted in the tubular guide member 61, and only the front end of the hub entrance boss 71c enters the rear end of the cylindrical side wall 25. Inasmuch as the needle hub 22 and the injection needle attachment 5 are not in engagement with each other, the injection needle 2 can easily be separated from the injection needle attachment 5. Since the injection needle attachment 5 and the needle hub 22 can be disengaged from each other and at the same time the cartridge piercing portion 21b can be pulled out of the sealing member 92 of the medicament solution cartridge 9, the injection needle 2 can quickly be removed from the injector 1.

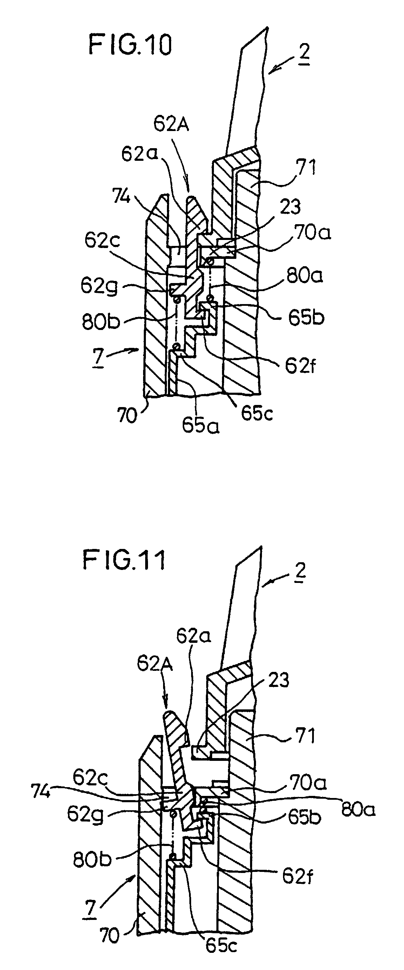

FIGS. 10 and 11 show an injector according to another embodiment of the present invention. Those parts of the injector shown in FIGS. 10 and 11 which are identical to those of the injector according to the previous embodiment are denoted by identical reference characters, and will not be described in detail below.

In the embodiment shown in FIGS. 10 and 11, the injector has a modified hub holder. In the previous embodiment, each of the hub holder fingers 62 has a lower end integrally formed with the cylindrical base 65. According to the embodiment shown in FIGS. 10 and 11, however, the hub holder comprises a cylindrical base 65a and a plurality of hub holder fingers 62A which are separate from the cylindrical base 65a. The cylindrical base 65a has on an upper end thereof a flange 65b extending upwardly, and a helical spring 80a is disposed between the planar panel 70a and the flange 65b. Each of the hub holder fingers 62A has on a lower end thereof an inwardly projecting tooth 62f projecting radially inwardly into engagement with a lower surface of the flange 65b, and an outwardly projecting tooth 62g aligned with and projecting radially outwardly away from the inwardly projecting edge 62c. A helical spring 80b is disposed between a lower surface of the outwardly projecting tooth 62g and a maximum-diameter step 65c of the cylindrical base 65a.

When the flange engaging edges 62a of the hub holder fingers 62A engage the flange 23 of the injection needle 2 as shown in FIG. 10, the hub holder fingers 62A extend along the axis of the medicament solution cartridge 9, with the helical spring 80a extended and the helical spring 80b compressed.

When the outer tubular member 70 is lowered in order to separate the needle hub 22 from the medicament solution cartridge 9, the inwardly projecting edges 62c are engaged by the inner surfaces of the recesses 74, angularly moving the hub holder fingers 62A radially outwardly thereby to displace the flange engaging edges 62a out of engagement with the flange 23, as shown in FIG. 11. As a result, the needle hub 22 can be removed from the hub holder.

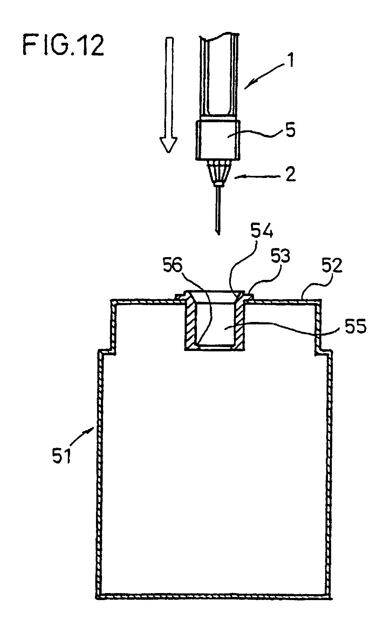

FIG. 12 shows an injection needle disposal container according to the present invention for holding a waste injection needle which is the used injection needle 2.

As shown in FIG. 12, the injection needle disposal container, generally denoted by 51, comprises a container housing 52 and a tubular injection needle separator 53 mounted on an upper panel of the container housing 52. The tubular injection needle separator 53 has a tapered guide surface 54 for guiding the injection needle attachment 5 of the injection needle 2 into the container housing 52, the tapered guide surface 54 being disposed around a central opening defined in the upper panel of the container housing 52 and progressively constricted downwardly, a cylindrical inner chamber 55 contiguous to and extending downwardly from the tapered guide surface 54 for housing at least the front end of the injection needle attachment 5, and an annular rib 56 projecting radially inwardly from a cylindrical wall of the cylindrical inner chamber 55 for limiting passage of the movable assembly 7 without substantially contacting the hub holder 6. The annular rib 56 has an inside diameter smaller than the outside diameter of the front end of the injection needle attachment 5, i.e., the front end of the outer tubular member 70 of the movable assembly 7, and is shaped so as not to contact the hub holder fingers 62 of the hub holder 6.

When the injection needle attachment 5 of the injector 1 is inserted into the tubular injection needle separator 53, the injection needle attachment 5 abuts against the rib 56. When the injector 1 is further pushed downwardly, only the movable assembly 7 of the injection needle attachment 5 remains held in the tubular injection needle separator 53, and the other parts of the injection needle attachment 5 are continuously pushed downwardly. Therefore, the movable assembly 7 is relatively moved backward toward the rear end of the injector casing, releasing the injection needle 2 from the injection needle attachment 5. The released injection needle 2 is then separated from the injector 1 and drops into the container housing 52 due to gravity and vibrations produced upon backward movement of the movable assembly 7 toward the rear end of the injector casing. The injection needle disposal container 51, with the above structure for assisting in moving the movable assembly 7 toward the rear end of the injector casing, permits the user to separate and discard the used injection needle 2 without touching the injection needle 2.

The injection needle disposal container 51 is shown as being of an integral unitary structure. However, it may comprise a container housing having an upper opening and a separate lid attached to close the upper opening and having the tubular injection needle separator 53. The rib 56 may not be of an annular shape, but may comprise a plurality of spaced teeth.

As described above, an injector according to the present invention comprises a medicament solution cartridge container for housing a medicament solution cartridge which has a gasket therein, a plunger mounted on the medicament solution cartridge container for pushing the gasket in the medicament solution cartridge, an injection needle attachment mounted on the medicament solution cartridge container, and an injection needle detachably mounted on the injection needle attachment, the injection needle comprising a hollow needle which has a piercing portion on a front end thereof and a cartridge piercing portion on a rear end thereof, and a needle hub fixedly mounted on an intermediate portion of the hollow needle, the injection needle attachment comprising a hub holder holding the needle hub, a movable assembly movable toward a rear end of the injector, and a hub attaching and detaching mechanism for releasing the needle hub from the hub holder when the movable assembly moves toward the rear end of the injector and holding the needle hub with the hub holder when the movable assembly moves toward a front end of the injector. The injector allows an injection needle of double-headed design to be easily attached thereto and detached therefrom without relying on threaded engagement or disengagement between the needle hub and the hub holder.

An injection needle according to the present invention comprises a hollow needle having front and rear pointed ends and a needle hub fixedly mounted on an intermediate portion of the hollow needle and having an open rear end, the needle hub having a hub rotation stop for contacting a needle rotation stop on an injection needle attachment of an injector to which the injection needle is to be attached. The injection needle, which is attached to an injector without threaded engagement therewith, is prevented from rotating with respect to the injector. Therefore, the user finds it easy to administer injections using the injection needle.

An injection needle disposal container for receiving an injection needle according to the present invention comprises a container housing and a tubular injection needle separator mounted on an upper panel of the container housing, the tubular injection needle separator having a guide surface for guiding the injection needle attachment of the injection needle into the container housing, an inner chamber extending downwardly from the guide surface for housing at least a front end of the injection needle attachment, and a rib projecting radially inwardly from an inner wall surface of the cylindrical inner chamber for limiting passage of a movable assembly of the injection needle without substantially contacting a hub holder of the injection needle. When the user pushes the injection needle attachment of the injector with an injection needle attached thereto into the tubular injection needle separator, the injection needle disposal container removes the injection needle easily from the injector and simultaneously holds the removed injection needle without letting the user touch the injection needle.

Although certain preferred embodiments of the present invention have been shown and described in detail, it should be understood that various changes and modifications may be made therein without departing from the scope of the appended claims.

## Claims

1. An injector (1) comprising:
a medicament solution cartridge container (3) for housing a medicament solution cartridge (9) which has a gasket (91) therein;
a plunger (4) mounted on said medicament solution cartridge container (3) for pushing the gasket (91) in the medicament solution cartridge (9); and
an injection needle attachment (5) mounted on said medicament solution cartridge container (3), on which an injection needle (2) detachably is mounted, said injection needle (2) comprising a hollow needle (21) which has a piercing portion (21a) on a front end thereof and a cartridge piercing portion (21b) on a rear end thereof, and a needle hub (22) fixedly mounted on an intermediate portion of said hollow needle (21);
said injection needle attachment (5) comprising a hub holder (6) holding said needle hub (22), a movable assembly (7) movable toward a rear end of the injector (1), and a hub attaching and detaching mechanism (62, 74a) for releasing said needle hub (22) from said hub holder (6) when said movable assembly (7) moves toward the rear end of the injector (1) and holding said needle hub (22) with said hub holder (6) when said movable assembly (7) moves towards the injection needle (2);
**characterised in that** said movable assembly (7) houses said hub holder (6) therein and has a spring (8, 80a) therein which is compressed between said movable assembly (7) and said hub holder (6).

2. An injector (1) according to claim 1, wherein said hub holder (6) comprises a hub holder finger assembly (60) having a plurality of hub holder fingers (62) and a tubular guide member (61) for guiding the cartridge piercing portion (21b) of the hollow needle (21), said tubular guide member (61) being fixed to or integral with said hub holder finger assembly (60).

3. An injector (1) according to claim 1 or 2, wherein said movable assembly (7) comprises an outer tubular member (70) and an inner tubular member (71) coaxially disposed in said outer tubular member (70), said inner tubular member (71) having a hub entrance boss (71c) for entering said needle hub (22) to prevent the needle hub (22) from wobbling when said injection needle (2) is mounted on the injector (1), and at least one cartridge presser (73b) extending away from said hub entrance boss (71c) for depressing said medicament solution cartridge (9).

4. An injector (1) according to claim 2, wherein said hub holder fingers (62) are elastically deformable so as to be spread radially outwardly when said movable assembly (7) moves toward the rear end of the injector (1).

5. An injector (1) according to claim 2, wherein said needle hub (22) has a flange (23) on a rear end thereof, each of said hub holder fingers (62) having a hub guiding slanted surface (62b) disposed on a front end thereof and a flange engaging edge (62a) disposed rearwardly of said hub guiding slanted surface (62b) for engaging said flange (23) of said needle hub (22).

6. An injector (1) according to claim 5, wherein each of said hub holder fingers (62) further includes an inwardly projecting tooth (62f) disposed rearwardly of said flange engaging edge (62a) for engaging an inner surface (74a) of a recess (74) of said movable assembly (7) thereby to angularly move each of said hub holder fingers (62).

7. An injector (1) according to any one of claims 4 through 6, wherein said injector (1) has an injector casing (11) with said medicament solution cartridge container (3) disposed therein, said hub holder (6) further comprising a cylindrical base (65) engaging said injector casing (11), said hub holder fingers (62) being integrally formed with said cylindrical base (65), each of said hub holder fingers (62) having an elastically deformable neck (62d) joined to said cylindrical base (65) for allowing the hub holder fingers (62) to angularly move with respect to said cylindrical base (65).

8. An injector (1) according to any one of claims 4 through 6, wherein said injector (1) has an injector casing (11) with said medicament solution cartridge container (3) disposed therein, said hub holder (6) further comprising a cylindrical base (65) engaging said injector casing (11), said hub holder fingers (62) being separate from said cylindrical base (65) and angularly movable with respect to said cylindrical base (65).

9. An injector (1) according to claim 8, further comprising a resilient member (80a) interposed between said cylindrical base (65) and a planar panel (70a) of said movable assembly (7) for normally biasing said movable assembly (7) toward the front end of the injector (1).

10. An injector (1) according to claim 9, wherein each of said hub holder fingers (62) has an inwardly projecting tooth (62f) on a rear end thereof for engaging a flange (65b) on a front end of said cylindrical base (65).

11. An injector (1) according to claim 8 or 9, wherein each of said hub holder fingers (62) has an outwardly projecting tooth (62g), further comprising a helical spring (80b) disposed between said outwardly projecting tooth (62g) and said cylindrical base (65).

12. An injector (1) according to any one of claims 1 through 11, wherein said injection needle attachment (5) has a needle rotation stop (72) for contacting said needle hub (22) to prevent said injection needle (2) from being rotated when the injection needle (2) is mounted on the injector (1).

13. An injector (1) according to claim 12, wherein said needle rotation stop (72) comprises a plurality of ribs (72) engageable with a rear end of said needle hub (22), said ribs (72) being mounted on a surface of a planar panel (70a) substantially perpendicularly to an axis of said movable assembly (7) for contact with said needle hub (22) when said injection needle (2) is mounted on the injector (1).

## Patentansprüche

1. Ein Injektor (1) umfassend:
einen Patronen-Behälter (3), um eine Patrone (9) für medizinische Lösungen aufzunehmen, in der sich ein Dichtungsring (91) befindet;
einen Ventilkolben (4), der an besagtem Patronen-Behälter (3) montiert ist, um den Dichtungsring (91) in der Patrone (9) für medizinische Lösungen zu verschieben; und
eine Injektionsnadel-Befestigung (5), die auf besagtem Patronen-Behälter (3) montiert ist, auf welchem eine Injektionsnadel (2) abtrennbar montiert ist, besagte Injektionsnadel (2) umfasst eine hohle Nadel (21), die an einem vorderen Ende davon einen stechenden Bereich (21a) und die an einem hinteren Ende davon einen eine Patrone anstechenden Bereich (21 b) hat, und eine Nadelhülse (22), die fest auf einem dazwischenliegenden Bereich besagter hohlen Nadel (21) montiert ist;
besagte Injektionsnadel-Befestigung (5) umfasst eine Hülsen-Halterung(6), um besagte Nadelhülse (22) zu halten, eine bewegliche Vorrichtung (7), die zu einem hinteren Ende des Injektors (1) bewegbar ist und einen Befestigungs- und Lösungsmechanismus (62, 74a) für die Hülse, um besagte Nadelhülse (22) aus besagter Hülsen-Halterung (6) zu lösen, wenn besagte bewegliche Vorrichtung (7) sich zu dem hinteren Ende des Injektors (1) bewegt, und um besagte Nadelhülse (22) mit besagter Hülsen-Halterung (6) zu halten, wenn besagte bewegliche Vorrichtung (7) sich in Richtung der Injektionsnadel (2) bewegt;
**dadurch gekennzeichnet,**
**dass** besagte bewegliche Vorrichtung (7) besagte Hülsen-Halterung (6) in sich aufnimmt und innen eine Feder (8, 80a) hat, die zwischen besagter beweglicher Vorrichtung (7) und besagter Hülsen-Halterung (6) zusammengedrückt ist.

2. Ein Injektor (1) nach Anspruch 1, bei welchem besagte Hülsen-Halterung (6) einen fingerartigen Aufbau (60) mit einer Mehrzahl von fingerartigen Hülsenhaltern (62) und ein rohrförmiges Führungsstück (61) zur Führung des die Patrone anstechenden Bereichs (21b) der hohlen Nadel (21) umfasst, wobei besagtes rohrförmiges Führungsstück (61) an besagtem fingerartigen Aufbau (60) befestigt oder integral mit ihm ausgeführt ist.

3. Ein Injektor (1) nach Anspruch 1 oder 2, bei welchem besagte bewegliche Vorrichtung (7) ein rohrförmiges Außenstück (70) und ein koaxial an besagtem rohrförmigen Außenstück (70) montiertes rohrförmiges Innenstück (71) umfasst, wobei besagtes rohrförmiges Innenstück (71) ein Hülsen-Einsatzstück (71c) hat, um in besagte Nadelhülse (22) zur Verhinderung des Wackelns der Nadelhülse (22) einzudringen, wenn besagte Injektionsnadel (2) in den Injektor (1) eingebaut ist, und zumindest eine Patronenpressvorrichtung (73b), die sich von besagtem Hülsen-Einsatzstück (71 c) wegerstreckt, um besagte Patrone (9) für medizinische Lösungen herunterzudrücken.

4. Ein Injektor (1) nach Anspruch 2, bei welchem besagte fingerartige Hülsenhalter (62) elastisch verbiegbar sind, so dass sie radial nach außen gespreizt werden, wenn besagte bewegliche Vorrichtung (7) sich zu dem hinteren Ende des Injektors (7) bewegt.

5. Ein Injektor (1) nach Anspruch 2, bei welchem besagte Nadelhülse (22) an einem hinteren Ende davon einen Flansch (23) hat, wobei jeder der besagten fingerartigen Hülsenhalter (62) eine schräge Hülsen-Führungsfläche (62b) hat, die sich an dessen oberen Ende befindet, und eine den Flansch beaufschlagende Kante (62a), die sich unterhalb besagter schräger Hülsen-Führungsfläche (62b) befindet, um besagten Flansch (23) besagter Nadelhülse (22) zu beaufschlagen.

6. Ein Injektor (1) nach Anspruch 5, bei welchem jeder der besagten fingerartigen Hülsenhalter (62) weiterhin einen nach innen vorstehenden Zahn (62f) umfasst, der sich unterhalb besagter den Flansch beaufschlagender Kante (62a) befindet, um eine Innenfläche (74a) eines Einschnittes (74) besagter beweglicher Vorrichtung (7) zu beaufschlagen, und dabei jeden besagten fingerartigen Hülsenhalter (62) um einen Winkel zu verschwenken.

7. Ein Injektor (1) nach einem der Ansprüche 4 bis 6, bei welchem besagter Injektor (1) ein Injektorgehäuse (11) hat, in dem besagter Patronen-Behälter (3) aufgenommen ist, wobei besagte Hülsen-Halterung (6) weiterhin eine zylindrische Basis (65) umfasst, die besagtes Injektorgehäuse (11) beaufschlagt, wobei besagte fingerartige Hülsenhalter (62) mit besagter zylindrischer Basis (65) integral ausgebildet sind, wobei jeder der besagten fingerartigen Hülsenhalter (62) einen elastisch deformierbaren Hals (62d) hat, der mit besagter zylindrischer Basis (65) verbunden ist, um es den fingerartigen Hülsenhaltern (62) zu erlauben, sich in Hinsicht auf besagte zylindrische Basis (65) um einen Winkel zu verschwenken.

8. Ein Injektor (1) nach einem der Ansprüche 4 bis 6, bei welchem besagter Injektor (1) ein Injektorgehäuse (11) hat, in dem besagter Patronen-Behälter (3) aufgenommen ist, wobei besagte Hülsen-Halterung (6) weiterhin eine zylindrische Basis (65) umfasst, die besagtes Injektorgehäuse (11) beaufschlagt, wobei besagte fingerartige Hülsenhalter (62) von besagter zylindrischer Basis (65) getrennt sind und in Hinsicht auf besagte zylindrische Basis (65) um einen Winkel verschwenkbar sind.

9. Ein Injektor (1) nach Anspruch 8, welcher weiterhin ein federndes Element (80a) umfasst, das sich zwischen besagter zylindrischer Basis (65) und einer planen Scheibe (70a) besagter beweglicher Vorrichtung (7) befindet, um besagte bewegliche Vorrichtung (7) zum vorderen Ende des Injektors (1) normal zu stützen.

10. Ein Injektor (1) nach Anspruch 9, bei welchem jeder der besagten fingerartigen Hülsenhalter (62) auf einem unteren Ende davon einen nach innen vorstehenden Zahn (62f) hat, um einen Flansch (65b) an einem vorderen Ende besagter zylindrischer Basis (65) zu beaufschlagen.

11. Ein Injektor (1) nach Anspruch 8 oder 9, bei welchem jeder der besagten fingerartigen Hülsenhalter (62) einen nach außen vorstehenden Zahn (62g) hat und weiterhin eine Schraubenfeder (80b) umfasst, die sich zwischen besagtem nach außen vorstehenden Zahn (62g) und besagter zylindrischer Basis (65) befindet.

12. Ein Injektor (1) nach einem der Ansprüche 1 bis 11, bei welchem besagte Injektionsnadel-Befestigung (5) eine Nadelrotationssperre (72) hat, um besagte Nadelhülse (22) zu kontaktieren, um besagte Injektionsnadel (2) am Drehen zu hindern, wenn die Injektionsnadel (2) auf den Injektor (1) montiert ist.

13. Ein Injektor (1) nach Anspruch 12, bei welchem besagte Nadelrotationssperre (72) eine Mehrzahl von Rippen (72) umfasst, die mit einem hinteren Ende besagter Nadelhülse (22) beaufschlagbar sind, wobei besagte Rippen (72) auf einer Oberfläche einer planen Scheibe (70a) im Wesentlichen senkrecht zu einer Achse der besagten beweglichen Vorrichtung (7) angebracht sind, um besagte Nadelhülse (22) zu kontaktieren, wenn besagte Injektionsnadel (2) auf den Injektor (1) montiert ist.

## Revendications

1. Injecteur (1) comportant :
un réceptacle de cartouche de solution de médicament (3) destinée à renfermer une cartouche de solution de médicament (9) qui a une garniture d'étanchéité (91) ;
un piston (4) monté sur ledit réceptacle de cartouche de solution de médicament (3) afin de pousser la garniture d'étanchéité (91) dans la cartouche de solution de médicament (9); et
une fixation d'aiguille d'injection (5) montée sur ledit réceptacle de cartouche de solution de médicament (3), sur laquelle une aiguille d'injection (2) est montée de façon démontable, ladite aiguille d'injection (2) comportant une aiguille creuse (21) qui a une partie de perçage (21a) sur une extrémité avant et une partie de perçage de cartouche (21b) sur une extrémité arrière, et un embout d'aiguille (22) monté de façon fixe sur une partie intermédiaire de ladite aiguille creuse (21);
ladite fixation d'aiguille d'injection (5) comportant un support d'embout (6) maintenant ledit embout d'aiguille (22), un ensemble mobile (7) mobile vers une extrémité arrière de l'injecteur (1), et un mécanisme de fixation et de démontage d'embout (62, 74) destiné à libérer ledit embout d'aiguille (22) dudit support d'embout (6) lorsque ledit ensemble mobile (7) se déplace vers l'extrémité arrière de l'injecteur (1) et à maintenir ledit embout d'aiguille (22) avec ledit support d'embout (6) lorsque ledit ensemble mobile (7) se déplace vers l'aiguille d'injection (2);
**caractérisé en ce que** ledit ensemble mobile (7) renferme ledit support d'embout (6) et possède un ressort (8, 80a) qui est comprimé entre ledit ensemble mobile (7) et ledit support d'embout (6).

2. Injecteur (1) selon la revendication 1, dans lequel ledit support d'embout (6) comporte un ensemble de doigts de support d'embout (60) ayant plusieurs doigts de support d'embout (62) et un élément de guidage tubulaire (61) afin de guider la partie de perçage de cartouche (21b) de l'aiguille creuse (21), ledit élément de guidage tubulaire (61) étant fixé sur ou faisant partie intégrante dudit ensemble de doigts de support d'embout (60).

3. Injecteur (1) selon la revendication 1 ou 2, dans lequel ledit ensemble mobile (7) comporte un élément tubulaire extérieur (70) et un élément tubulaire intérieur (71) disposé coaxialement dans ledit élément tubulaire extérieur (70), ledit élément tubulaire intérieur (71) ayant un bossage d'entrée d'embout (71c) destiné à entrer dans ledit embout d'aiguille (22) afin d'empêcher l'embout d'aiguille (22) d'osciller lorsque ladite aiguille d'injection (2) est montée sur l'injecteur (1), et au moins un poussoir de cartouche (73b) qui s'étend à l'écart dudit bossage d'entrée d'embout (71c) afin d'enfoncer ladite cartouche de solution de médicament (9).

4. Injecteur (1) selon la revendication 2, dans lequel lesdits doigts de support d'embout (62) sont déformables élastiquement de façon à être écartés radialement vers l'extérieur lorsque ledit ensemble mobile (7) se déplace vers l'extrémité arrière de l'injecteur (1).

5. Injecteur (1) selon la revendication 2, dans lequel ledit embout d'aiguille (22) a un rebord (23) sur une extrémité arrière, chacun desdits doigts de support d'embout (62) ayant une surface inclinée de guidage d'embout (62b) disposée sur une extrémité avant et un bord d'engagement de rebord (62a) disposé vers l'arrière de ladite surface inclinée de guidage d'embout (62b) afin d'engager ledit rebord (23) dudit embout d'aiguille (22).

6. Injecteur (1) selon la revendication 5, dans lequel chacun desdits doigts de support d'embout (62) comprend en outré une dent qui dépasse vers l'intérieur (62f) disposée vers l'arrière dudit bord d'engagement de rebord (62a) afin d'engager une surface interne (74a) d'un renfoncement (74) dudit ensemble mobile (7) de façon à déplacer ainsi angulairement chacun desdits doigts de support d'embout (62).

7. Injecteur (1) selon l'une quelconque des revendications 4 à 6, dans lequel ledit injecteur (1) possède un boîtier d'injecteur (11) avec ledit réceptacle de cartouche de solution de médicament (3) disposé dedans, ledit support d'embout (6) comportant en outre une base cylindrique (65) qui engage ledit boîtier d'injecteur (11), lesdits doigts de support d'embout (62) étant formés intégralement avec ladite base cylindrique (65), chacun desdits doigts de support d'embout (62) ayant un col élastiquement déformable (62d) relié à ladite base cylindrique (65) afin de permettre aux doigts de support d'embout (62) de se déplacer angulairement par rapport à ladite base cylindrique (65).

8. Injecteur (1) selon l'une quelconque des revendications 4 à 6, dans lequel ledit injecteur (1) a un boîtier d'injecteur (11) avec ledit réceptacle de cartouche de solution de médicament (3) disposé dedans, ledit support d'embout (6) comportant en outre une base cylindrique (65) qui engage ledit boîtier d'injecteur (11), lesdits doigts de support d'embout (62) étant séparés de ladite base cylindrique (65) et mobiles angulairement par rapport à ladite base cylindrique (65).

9. Injecteur (1) selon la revendication 8, comportant en outre un élément élastique (80a) interposé entre ladite base cylindrique (65) et un panneau plan (70a) dudit ensemble mobile (7) afin de rappeler normalement ledit ensemble mobile (7) vers l'extrémité avant de l'injecteur (1).

10. Injecteur (1) selon la revendication 9, dans lequel chacun desdits doigts de support d'embout (62) a une dent qui dépasse vers l'intérieur (62f) sur une extrémité arrière afin d'engager un rebord (65b) sur une extrémité avant de ladite base cylindrique (65).

11. Injecteur (1) selon la revendication 8 ou 9, dans lequel chacun desdits doigts de support d'embout (62) a une dent qui dépasse vers l'extérieur (62g), comportant en outre un ressort hélicoïdal (80b) disposée entre ladite dent qui dépasse vers l'extérieur (62g) et ladite base cylindrique (65).

12. Injecteur (1) selon l'une quelconque des revendications 1 à 11, dans lequel ladite fixation d'aiguille d'injection (5) a une butée de rotation d'aiguille (72) destinée à venir en contact avec ledit embout d'aiguille (22) de façon à empêcher ladite aiguille d'injection (2) d'être entraînée en rotation lorsque l'aiguille d'injection (2) est montée sur l'injecteur (1).

13. Injecteur (1) selon la revendication 12, dans lequel ladite butée de rotation d'aiguille (72) comporte plusieurs nervures (72) pouvant être engagées avec une extrémité arrière dudit embout d'aiguille (22), lesdites nervures (72) étant montées sur une surface d'un panneau plan (70a) sensiblement perpendiculaire à un axe dudit ensemble mobile (7) afin de venir en contact avec ledit embout d'aiguille (22) lorsque ladite aiguille d'injection (2) est montée sur l'injecteur (1).
